# EUROPÄISCHE PATENTSCHRIFT

(11) **EP 0 520 322 B1**
(45) Veröffentlichungstag und Bekanntmachung des Hinweises auf die Patenterteilung: **30.08.1995**
(21) Anmeldenummer: 92110310.7
(22) Anmeldetag: 18.06.1992
(51) Int. Cl.: G01N 21/90, G01N 21/35, B07C 5/34, G01J 3/28

(54) **Verfahren und Vorrichtung zum Untersuchen des gasförmigen Inhalts retournierter Getränkeflaschen**
Method and apparatus for investigating the gas content of returned drink bottles
Procédé et appareil de contrôle de la teneur en gaz des bouteilles consignées

(30) Priorität: 28.06.1991 DE 4121429
(43) Veröffentlichungstag der Anmeldung: 30.12.1992
(73) Patentinhaber: Krieg, Gunther, Prof.Dr.Ing., D-76227 Karlsruhe (DE)
(72) Erfinder: Koukolitschek, Karl, Dipl.-Ing., W-7500 Karlsruhe 21 (DE); Krieg, Gunther, Prof. Dr. Ing., W-7500 Karlsruhe 41 (DE); Maier, Wilfried, W-7519 Sulzfeld (DE)
(74) Vertreter: Dipl.-Ing. Heiner Lichti Dipl.-Phys. Dr.rer.nat. Jost Lempert Dipl.-Ing. Hartmut Lasch

(56) Entgegenhaltungen:
- WO-A-88/00862
- DE-A- 4 038 993
- US-A- 3 321 954
- US-A- 4 858 767
- TECHNISCHES MESSEN TM. Bd. 58, Nr. 3, März 1991, MUNCHEN DE Seiten 122 - 127;J.STAAB: 'Industrielle Gasanalyse'
- APPLIED SPECTROSCOPY. Bd. 43, Nr. 1, Januar 1989, BALTIMORE US Seiten 27 - 32;D.M. MAYES ET AL: 'A photodiode-array based near-infrared spectrophotometer for the 600-1100 nm wavelength region'
- ANALYTICAL CHEMISTRY. Bd. 62, Nr. 10, Mai 1990, COLUMBUS US Seiten 1034 - 1043; J.J. SULLIVAN ET AL: 'Characterization of a computerized photodiode array spectrometer for gas chromatography-atomic emission spectrometry'
- APPLIED SPECTROSCOPY. Bd. 44, Nr. 5, Juni 1990, BALTIMORE US Seiten 822 - 825; H.H. RICHARDSON ET AL: 'A novel infrared spectrometer using a linear array detector'
- REVIEW OF SCIENTIFIC INSTRUMENTS. Bd. 60, Nr. 9, September 1989, NEW YORK US Seiten 2920 - 2923; M.A. YOUNG et al.: "Signal processing for an infrared array detector"

## Beschreibung

Die Erfindung betrifft ein Verfahren nach dem Oberbegriff des Anspruchs 1 und eine Vorrichtung nach dem Oberbegriff des Anspruchs 19.

Die qualitative und quantitative Detektion und Identifikation von Schadstoffen in Mehrweg-Getränkeflaschen, wie z. B. von Holzschutzmitteln, Allzweckreinigern, Ölen, Benzin, Pflanzenschutzmitteln und sonstigen Giftstoffen, ist aus Gründen der Produkthaftung, des Schutzes menschlichen Lebens, der Geschmacksqualität von Getränken, des Umweltschutzes und der Problematik der Entsorgung von Giftstoffen, ein wichtiges Problem. Diese Problematik liegt vor allem bei Kunststoff-Mehrwegflaschen, z. B. aus Polyethylen, Polycarbonat vor, da infolge hoher Diffusionskoeffizienten die Schadstoffe in die Flaschenwandung hineindiffundieren. Die Schadstoffe werden daher im Waschprozeß nicht vollständig entfernt und können nach Wiederbefüllung der Flasche in das jeweilige Getränk zurückdiffundieren und dessen Geschmacksqualität negativ beeinflussen.

Die US-A-3 321 954 zeigt ein grundsätzlich gattungsgemäßes Verfahren, bei dem eine aus Flaschen entnommene Gasprobe mittels eines Flammen-Ionisations-Detektors untersucht wird.

Es ist weiter bekannt, unter Einsatz von Photoionisationsdetektoren, welche nach Entnahme einer Probe aus dem Gasraum der jeweiligen Flasche diese Probe mit ultravioletter Strahlung teilweise ionisieren, und durch Messung des in einem elektrischen Feld resultierenden Ionenstroms zu versuchen, auf die etwaige Anwesenheit von Schadstoffen zu schließen. Da durch derartige Ionisationsdetektoren auch ungefährliche Gase, wie z. B. Wasserdampf, Kohlendioxid, Methan usw., erfaßt werden, muß die Ionisierungsenergie des ultravioletten Lichts so niedrig gewählt werden, daß diese Stoffe nicht angezeigt werden. Letzteres hat jedoch zur Folge, daß gefährliche Schadstoffe, wie z. B. Benzin, Öle, Diesel, Isopropanol etc. sich ebenfalls einer Erkennung entziehen.

Ferner sind aufgrund der in der Getränkeindustrie üblichen hohen Flaschenleistungen von bis zu 50.000 Flaschen pro Stunde und den daraus resultierenden kurzen Meßzeiten pro Flasche Vielfach-Sensoranordnungen mit mehr als 20 Sensorsystemen erforderlich, was zu unvertretbar hohen Kosten für das Gesamtsystem führt.

In der US-Patentschrift Nr. 4,858,767 wird auf den Einsatz von Massenspektrometern hingewiesen. Derartige Systeme sind jedoch nur bedingt zur Lösung des Problems geeignet, da die zur Massenselektion erforderliche Ionisierung notgedrungen auch zu einer Fragmentierung der Moleküle führt, so daß die Ionisationsprodukte aus anderen Molekülverbindungen als die eigentlichen, ursprünglich zu analysierenden Stoffen bestehen. Letzteres tritt verstärkt bei in Getränken sehr häufig vertretenen großen Molekülen auf, so daß die Messung keinen eindeutigen Schluß auf die Giftigkeit oder Ungiftigkeit des jeweiligen Gasgemisches zuläßt. Bei beiden Verfahren kann es zu einer Verschmutzung des Sensors kommen, die seine Effektivität reduziert.

Nach der US-Patentschrift Nr. 4,858,768 wird aufgrund eines Ionisationsverfahrens eine Flasche schon dann aus dem Abfüllprozeß ausgeschieden, wenn Abweichungen zum reinen Getränk vorliegen. Dies ist jedoch aus wirtschaftlichen Gründen nicht akzeptabel, da Abweichungen bereits dann vorliegen, wenn ungiftige Stoffe dem Getränk zugemischt werden, so daß auch als unbedenklich geltende Flaschen aus dem Prozeß ausgeschieden werden.

Es wurde allgemein schon vorgeschlagen, den gasförmigen Inhalt retournierter Getränkeflaschen auch mittels Infrarotspektroskopie zu untersuchen. Herkömmliche Infrarotspektroskopie weist in der Regel nicht die gewünschte Auflösung auf und ist grundsätzlich zeitaufwendig, wobei ein hoher Zeitbedarf insbesondere dadurch bedingt ist, daß mangels hoher Auslösung ein sehr großer Wellenlängenbereich erfaßt werden muß.

Die WO-88/0862 schlägt allgemein IR-Spektroskopie vor, ohne die Auswertung zu spezifizieren. Neben den genannten Nachteile, ist insbesondere auch eine Analyse des Gesamtspektrums zu (zeit-)aufwendig oder zu teuer. Soweit Spektroskopie im nahen Infrarot zwischen 1,1 »m und 2,5 »m vorgeschlagen wird, hat sich einerseits herausgestellt, daß dieser Bereich nicht kritisch genug ist, d. h. die Spektren in diesem Bereich - da nicht molekülspezifisch - nicht aussagekräftig sind. Ferner ist die Nachweisempfindlichkeit im nahen Infrarot äußerst gering. Andererseits ist die notwendige Auswertung mittels Regressionsanalyse zeitaufwendig.

Die Erfassung erfolgt zwar bei einem Fourier-Transformations-Spektrometer schneller, da zeitgleich. Die Auswertung erfordert aber - für den vorgesehenen Einsatzzweck nicht vertretbar - teure Rechner und ist mit Spektrenauswertung und Vergleich mit Musterspektren für die o.a. herkömmliche Getränkeabfüllanlagen zu langsam.

Aus J. Staab "Industrielle Gasanalyse" in Technisches Messen TM, Bd. 58, Nr. 3, März 1991, München, S. 122-127, sind grundsätzlich Arrays von Photoelementen bekannt, mit denen eine gleichzeitige Detektion in einem großen Spektralbereich möglich ist. H.H. Richardson et al. "A novel infrared spectrometer using a linear array detector" in Applied Spectroscopy, Bd. 44, Nr. 5, Juni 1990, Baltimore, S. 822-825, schlägt ein Spektrometer mit einem Photodioden-Array zum Empfang eines IR-Spektrums im Bereich von 2400-2650 cm⁻¹ (entsprechend 3,77-4,17 »m) vor.

Der Erfindung liegt die Aufgabe zugrunde, ein Verfahren und eine Vorrichtung mit hoher Auflösung zu schaffen, um die Stoffunterscheidung in einem begrenzten Wellenlängenbereich und mit einer kurzen Meßzeit durchführen zu können.

Erfindungsgemäß wird die genannte Aufgabe mit einem Verfahren nach dem Anspruch 1 und einer Vorrichtung nach dem Anspruch 19 gelöst.

Die Erfindung sieht damit die gleichzeitige Detektion in einem für die interessierenden Stoffe höchst relevanten, aber sehr engen Spektralbereich vor, so daß eine schnelle Erkennung der Stoffe mit hoher Empfindlichkeit ohne hohen Verarbeitungsaufwand möglich ist.

Erfindungsgemäß werden Giftstoffe enthaltende Flaschen zerstörungsfrei, d.h. ohne Fragmentierung der Giftstoffe erkannt und aus der Getränkeabfüllinie ausgeschieden und von solchen Flaschen unterschieden, die lediglich ungefährliche Gärprodukte enthalten, wie sie beispielsweise aus Resten von Süßgetränken während des Rücktransports oder der Lagerung von Leergut entstehen. Im Bereich der Brunnenindustrie wird durch die Erfindung darüber hinaus eine Unterscheidung und Selektion zwischen Leergutflaschen möglich, die mit Süßgetränken gefüllt waren und von solchen, die Mineralwasser enthielten durchgeführt, um zu verhindern, daß Süßgetränkeflaschen mit Mineralwasser befüllt werden, was zu Geschmacksbeeinträchtigungen führen würde, da nicht zu vermeiden ist, daß Aromastoffe von Süßgetränken aus der Flaschenwandung in das Mineralwasser hineindiffundieren.

Grundsätzlich kann vorgesehen sein, daß das Sortieren und Ausscheiden von Flaschen in zwei Wegen erfolgt, wobei Separierung dahingehend erfolgt, ob die Flaschen entweder Giftstoffe oder unästhetische Stoffe, wie Benzin, Öle, Diesel, Isopropanol, Urin od. dgl. enthalten oder aber an sich ungefährliche und/oder durch Reinigen weitgehend entfernbare Stoffe, wie Gärprodukte, Aromastoffe oder lediglich Luft und Mineralwasserdampf enthalten haben. Eine solche Separierung bietet sich dort an, wo die Flaschen mit Aromastoffen enthaltenen Flüssigkeiten, wie Limonaden und Colagetränken befüllt werden, die die Aromen dieser ungefährlichen Stoffe überdecken. In äußerst bevorzugter Ausgestaltung ist aber vorgesehen, daß die Flaschen in drei Wegen separiert werden, nämlich zunächst Giftstoffe oder unästhetische Stoffe; derartige Flaschen werden völlig ausgeschieden und vernichtet. Der weitere Weg kann Flaschen mit Gärprodukten und Aromastoffen enthalten. Diese Flaschen können, wie gesagt, einer erneuten Befüllung mit Aromastoffen enthaltenen Getränken zugeleitet werden. Den dritten Weg werden Flaschen zugeführt, die lediglich Mineralwasser-Dampf und die üblichen Luftanteile als gasförmige Stoffe enthalten; derartige Flaschen können der Befüllung durch Mineralwasser zugeführt werden. Einer solchen Befüllung werden Flaschen, die ungefährliche Gärprodukte oder Aromastoffe, wie auch von Getränken, enthalten haben, nicht zugeführt, da solche Restaromen den Geschmack von reinem Wasser, sei es stillem oder kohlesäurehaltigem Mineralwasser, überdecken und verfälschen können.

Gemäß einer bevorzugten Ausgestaltung wird vorzugsweise mit einer Auflösung von besser als 0,2 »m gearbeitet. In äußerst bevorzugter Ausgestaltung ist vorgesehen, daß die Detektorreihe einen Detektor auf 100 »m enthält und vorzugsweise der Abstand zwischen zwei Detektoren der Detektorreihe der Breite der Detektoren selbst entspricht. Um die Auflösung hier zu erhöhen, kann in bevorzugter Ausgestaltung weiterhin vorgesehen sein, daß die Detektorreihe um die Breite eines einzelnen Detektors der Reihe in Richtung derselben versetzt wird. Ein Hin- und Rückversatz ist ausreichend, wenn der lichtempfindliche Abstand zweier Detektoren der Detektorreihe nicht größer als die Breite der Detektoren selbst ist. Falls Detektorreihen mit lichtunempfindlichen Abständen gewählt werden, deren Breite größer als die der einzelnen Detektoren ist, so kommt ein mehrmaliger Versatz in einer Richtung in Frage, bis der bei der ersten Messung unempfindliche Bereich der Detektorreihe bei den weiteren nach Versatz durchgeführten Messungen vollständig abgedeckt ist.

In äußerst bevorzugter Ausgestaltung ist weiterhin vorgesehen, daß die Höhe der Detektoren senkrecht zur Erstreckungsrichtung der nebeneinander angeordneten Detektoren der Reihe größer ist als deren Breite und insbesondere ein zigfaches der Breite beträgt. Hierdurch kann die erforderliche Meßzeit wesentlich reduziert werden.

In weiterer bevorzugter Ausgestaltung ist vorgesehen, daß das die Spektralzerlegung bewirkende Element ein Gitter, insbesondere ein Reflexionsgitter und vorzugsweise ein Blazegitter ist. Hierbei handelt es sich um ein Gitter mit nebeneinander angeordneten, um einen bestimmten Winkel aus der Gitterebene geneigten Mikrospiegeln, wodurch die Maxima erster Ordnung, in denen eine Spektralzerlegung stattfindet, eine hinreichende und größere Intensität erhalten, als dies bei einem herkömmlichen Gitter der Fall ist. In einer anderen bevorzugten Ausgestaltung wird ein holographisches Gitter verwendet.

Zur Verminderung des Rauschens im Empfangsbereich, nämlich des Detektors und des elektronischen Rauschens, ist vorgesehen, daß der Detektor und die Elektronik gekühlt werden, wobei insbesondere eine Kühlung des Detektors mittels eines Peltierelementes erfolgen kann.

Zur Eliminierung von Umwelteinflüssen und verbleibendem Rauschens sieht eine bevorzugte Ausgestaltung vor, daß das ausgesandte Licht getaktet bzw. intensitätsmoduliert wird. Dies kann entweder, bei einem Plankschen Strahler geringer Wärmekapazität, wie einem Draht oder einer dünnen leitfähigen Schicht elektronisch oder aber bei trägen Lichtquellen, wie Infrarotdioden, durch einen mechanischen Chopper erfolgen, wobei die Signalauswertung durch einen Lock-In-Verstärker durchgeführt wird.

Hierbei zeigt bzw. zeigen:
- Figur 1: das zur Klassifizierung in verschiedene Kategorien skizzierte Gesamtsystem;
- Figur 2: ein Ausführungsbeispiel zur optoelektronischen Klassifizierung in verschiedene Kategorien, mit
- Figur 2a: einer detailierten Darstellung des optischen Aufbaus;
- Figur 3: ein Ausführungsbeispiel zur weiteren Steigerung der Flaschenleistung in Getränkeabfüllanlagen bei gleichzeitiger Aufrechterhaltung der Qualität der Klassifizierung;
- Figur 4: ein Spektrum von Raumluft, sowie Spektren von Süßgetränken, von Schadstoffen, von Gärprodukten;
- Figur 5: eine in den optischen Strahlengang einschwenkbare Testzelle zur Wellenlängekalibrierung der Detektorreihe; und
- Figur 6: ein Blockschaltschild der elektronischen Signalverarbeitung.

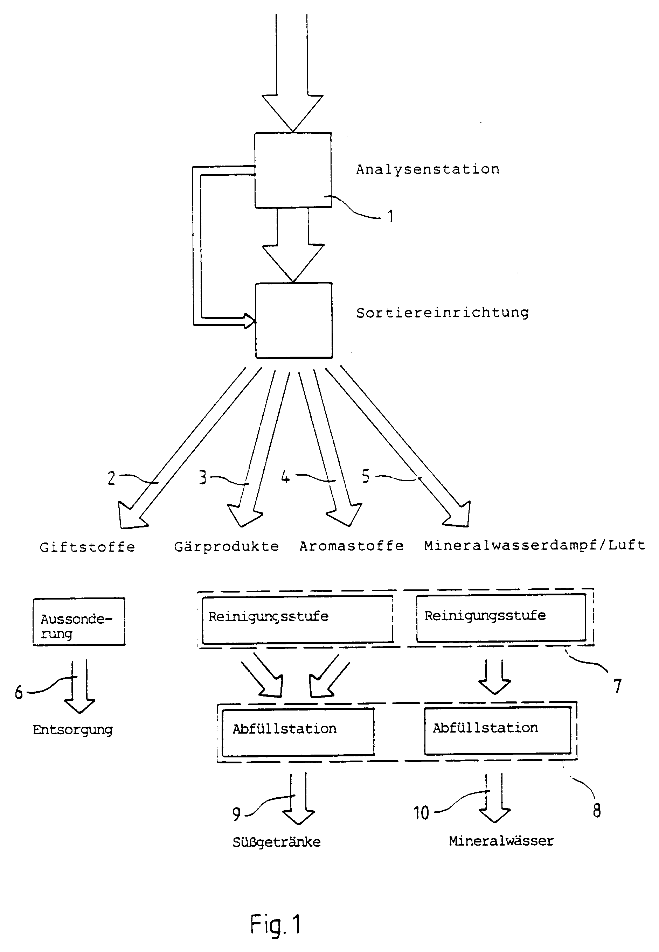

Entsprechend Figur 1 werden die Gasinhalte der Mehrwegflaschen in einer Analysenstation 1 zerstörungsfrei analysiert. Gemäß dem Ergebnis der on-line-Analyse werden die Flaschen entsprechend ihren Gasinhalten in Giftstoffe 2, Gärprodukte 3, Aromastoffe 4, Luft, Mineralwasserdampf 5 klassifiziert. Die Giftstoffe 2 enthaltenden Flaschen werden ausgesondert und automatisch einer Entsorgung zugeführt. Die übrigen Flaschen durchlaufen eine Reinigungsstufe 7 und gelangen anschließend in die Getränkeabfüllstationen 8. Dabei werden die Flaschen, welche Gärprodukte bzw. Aromastoffe enthielten, der Süßgetränkelinie 9 zugeführt, wohingegen die durch Luft, Wasserdampf 5 gekennzeichneten Flaschen in die Mineralwasserlinie 10 laufen.

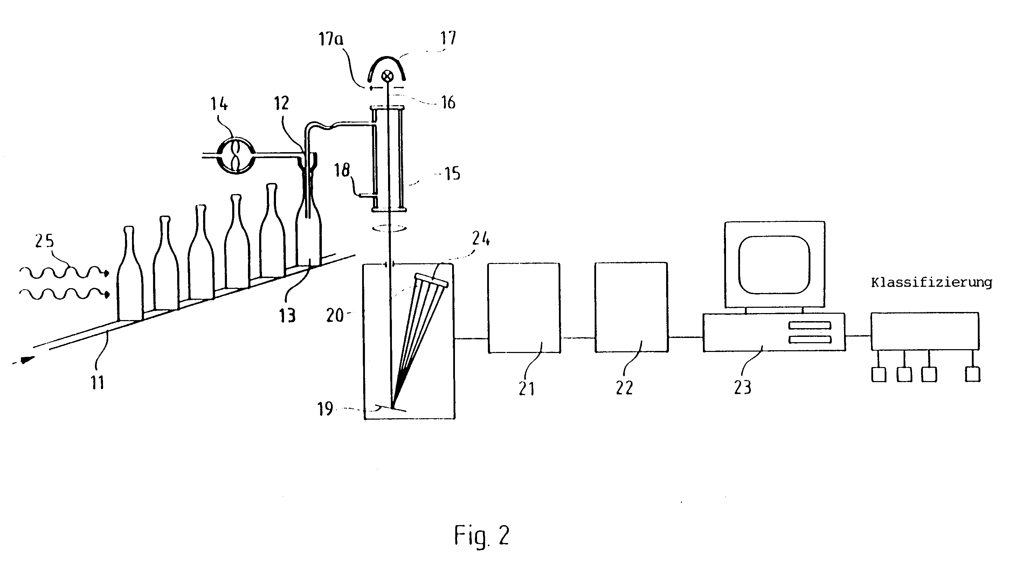

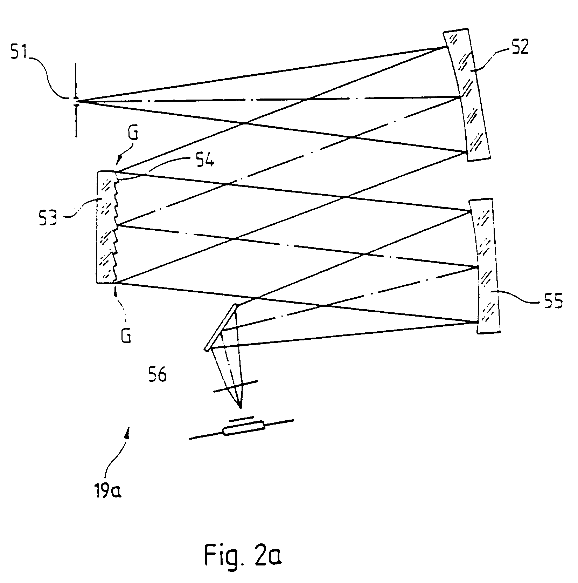

Gemäß Figur 2 werden die Flaschen mechanisch über eine Transportvorrichtung 11 dem Klassifizierungssystem zugeführt. Das Klassifizierungssystem besteht aus einer Hochgeschwindigkeitsprobenahme 12, die eine Gasprobe der jeweiligen Flasche 13 entnimmt, indem über eine Druckeinheit 14 ein Gasvolumen der Untersuchungskammer 15 zugeleitet wird. Alternativ kann die Pumpe 14 auch an Stutzen 18 angebracht werden, sodaß die Probenahme durch einen Saugvorgang erfolgt. Die Untersuchungskammer 15 wird von einem von einer IR-Lichtquelle erzeugten und durch einen Zerhacker (Chopper) 17a getakteten Strahl durchsetzt, der und anschließend von einer Dispersionseinheit 19 in seine Spektralanteile zerlegt wird. Die spektrale Verteilung wird von einer Vielzahl linear angeordneter Sensorelemente 20 erfaßt und von einer nachgeschaltenen Signalverarbeitung 21 mikroelektronisch weiterverarbeitet.

Die Figur 2a zeigt den in der Figur 2 nur pauschal angegebenen optischen Aufbau detailierter. Der aus der Küvette oder Untersuchungskammer 15 austretende Strahl tritt durch einen Eintrittsspalt 51 in das Spektrometer 19a ein und wird über einen ersten Hohlspiegel 52 parallelisiert (Abbildung ins Unendliche). Als Dispersionseinheiten 19 werden vorzugsweise optische Gitter oder Prismen eingesetzt. Der Parallelstrahl trifft im dargestellten Ausführungsbeispiel auf ein Spektral-Dispersionselement in Form eines sogenannten "Blazed"-Gitters 53, das gegenüber einem herkömmlichen Reflexionsgitter keine miteinander fluchtenden Spiegelflächen (mit nichtspiegelnden Bereichen dazwischen), sondern um einen Blazewinkel aus der Gitterebene G geneigte Spiegel 54 hat. Dadurch wird das auftreffende Licht mit einem endlichen Gangunterschied bevorzugt in eine Richtung reflektiert. Grundsätzlich läßt sich hierdurch für die Maxima 1. Ordnung der Spektrumzerlegung eine höhere Intensität erreichen als bei einem konventionellen Gitter, bei dem das nicht spektralzerlegte Maximum nullter Ordnung die überwiegenden Intensität enthält.

Der durch das Gitter 53 spektral zerlegte Strahl wird dann durch einen weiteren Hohlspiegel 55 über einen Umlenkspiegel 56 auf die mittels Peltierelementen 24 gekühlte Detektorreihe 20 mittels einer Linse L abgebildet. Durch diese Abbildungsoptik wird bei kompaktem Aufbau eine gute Abbildung des Eintrittspalts 51, insbesondere hinsichtlich seiner Breite, auf die Detektorreihe 20 (je nach "Farbe") erreicht.

Ein Signalprozessorsystem 48 ermittelt die jeweilige spektrale Verteilung und vergleicht sie mit den in einem Speicher 23 abgelegten Spektren, so daß eine eindeutige Identifizierung der jeweiligen Substanzen resultiert.

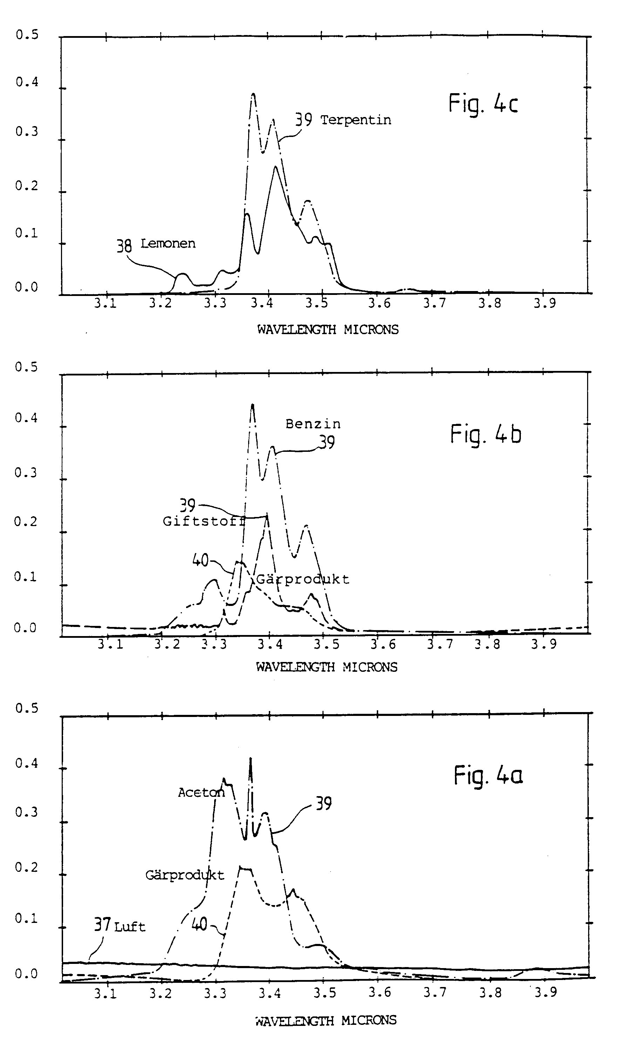

Als Sensorelemente 20 werden im Infrarot-Bereich Arrays von Photoelementen aus Silizium- oder Quecksilber-Cadmium-Tellurid(MCT)-Halbleiter-Verbindungen verwendet. Auch PbSe-Sensoren können eingesetzt werden. Die Einzelsensoren haben eine Breite in ihrer Erstreckungsrichtung im »m-Bereich von beispielsweise 50 »m. Die Lücke zwischen den Sensoren liegt vorzugsweise in der gleichen Größenordnung. Bei einer Lücke von 50 »m, also Detektorenabstand (Mitte zu Mitte) von 100 »m, weist eine Detektorreihe von 32 Detektorelementen eine Länge von 3,2 mm auf. Es hat sich herausgestellt, daß gerade der Spektralbereich zwischen 3,2 und 3,6 »m für den angestrebten Einsatzzweck einen äußerst charakteristischen Spektralausschnitt bildet, wie die Figuren 4a - 4c zeigen. Nach Spektralzerlegung wird dieser Spektralbereich auf die Detektorreihe von 3,2 mm und damit auf die 32 Detektorelemente abgebildet. Zur Erhöhung der Meßpunkte kann die Detektorreihe in bevorzugter Ausbildung gerade um die Hälfte des Abstandes von 100 »m, als um 50 »m (entsprechend der Lückenbreite) verschoben werden, wodurch auch das in diesem Bereich treffende Licht erfaßt wird.

Die Sensorelemente haben vorzugsweise eine ihre Breite beträchtlich überragende Länge (senkrecht zur Aneinanderreihung) von hier 1,5 mm, um bei der wegen der erforderlichen Auflösung geringen Intensität des auftreffenden Lichts hinreichenden Lichtmengen zu empfangen. Dies umso mehr als bei der verwendeten Optik zwar die Spaltbreite gut abgebildet wird, seine Länge aber verzerrt wird. Diese Detektoren werden zur Verbesserung der Strahlungsdetektion vorzugsweise mit 3-stufigen Peltier-Elementen 24 auf Temperaturen unterhalb -70 °C gekühlt. Die Stoffidentifikation erfolgt erfindungsgemäß derart, daß Spektren von Raumluft 37 gemäß Figur 4 von den aktuell gemessenen Spektren 38, 39, 40 von Süßgetränken, von Schadstoffen, von Gärprodukten subtrahiert werden und daß das Ergebnis der Subtraktion mit den im Speicher 23 abgelegten Spektren verglichen wird, sodaß eine Identifizierung der Schadstoffe bezüglich der jeweiligen Molekülverbindung als auch der Schadstoffkonzentration resultiert.

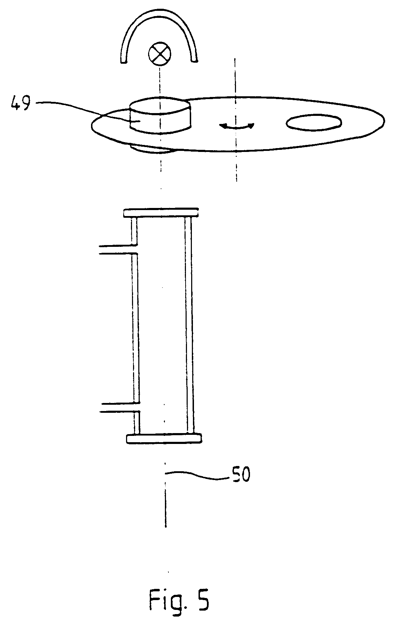

Um z. B. temperaturbedingte Änderungen in der Wellenlängenskala zu korrigieren, wird erfindungsgemäß analog zu Figur 5 eine mit Testgas, z. B. Methan, gefüllte Testzelle 49 zu gewissen Zeiten in den Strahlengang geschwenkt. Aus der physikalisch bekannten Lage der Spektrallinien des Testgases auf der Wellenlängenskala kann eine Neukalibrierung der jeweiligen Detektorelementposition, z. B. durch Interpolation, abgeleitet werden.

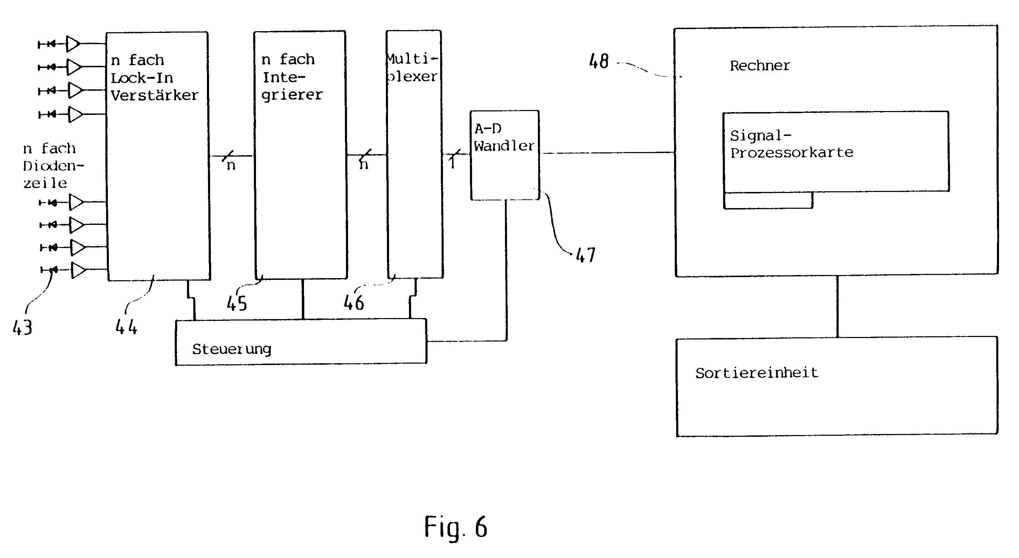

Die auf die Array-Detektorelemente 43, periodisch nach elektronsicher oder mechanischer Modulation auftreffende Strahlung unterschiedlicher Wellenlängenbereiche, wird gemäß Figur 6 durch n parallele Lock-in-Verstärker 44, die mit der Modulationseinrichtung synchronisiert sind, sowie durch n parallele, zeitgesteuerte Integratoren 45 weiterverarbeitet. Über einen Multiplexer 46 sowie einen A/D-Wandler 47, werden die Meßwerte dem zentralen Rechensystem 48 zur endgültigen Verarbeitung und zur Auslösung von Steuervorgängen zugeführt.

Um auch relativ komplizierte Spektren gemäß Figur 4 mit einer genügend hohen Anzahl von Meßpunktepaaren bei gleichzeitig vergleichsweise niedriger Anzahl von Einzeldetektoren (Pixel) in der Detektorreihe erfassen zu können, wird in einer Weiterbildung der Erfindung eine mikromechanische Verschiebung des Sensor-Arrays 20 vorgesehen, wobei die Verschiebung so gewählt wird, daß die Pixel Positionen einnehmen, die zwischen der ursprünglichen Lage von zwei Pixeln sich befinden. Vorzugsweise kann zur Verschiebung ein Piezo-Translator verwendet werden. In analoger Weise kann dieser Effekt auch durch leichtes Verkippen des Dispersionselementes 19 um eine Drehachse erreicht werden.

Zur Steigerung der Sensitivität der Stoffdetektion wird der Partialdruck der nachzuweisenden Stoffe vorzugsweise durch Einstrahlung von Infrarotlicht 25 bzw. von Mikrowellenstrahlung gemäß Figur 2 erhöht, indem sich die in der Flaschenwandung absorbierten Moleküle durch Energiezufuhr aus der Flaschenwandung ablösen und in den Gasraum hineindiffundieren.

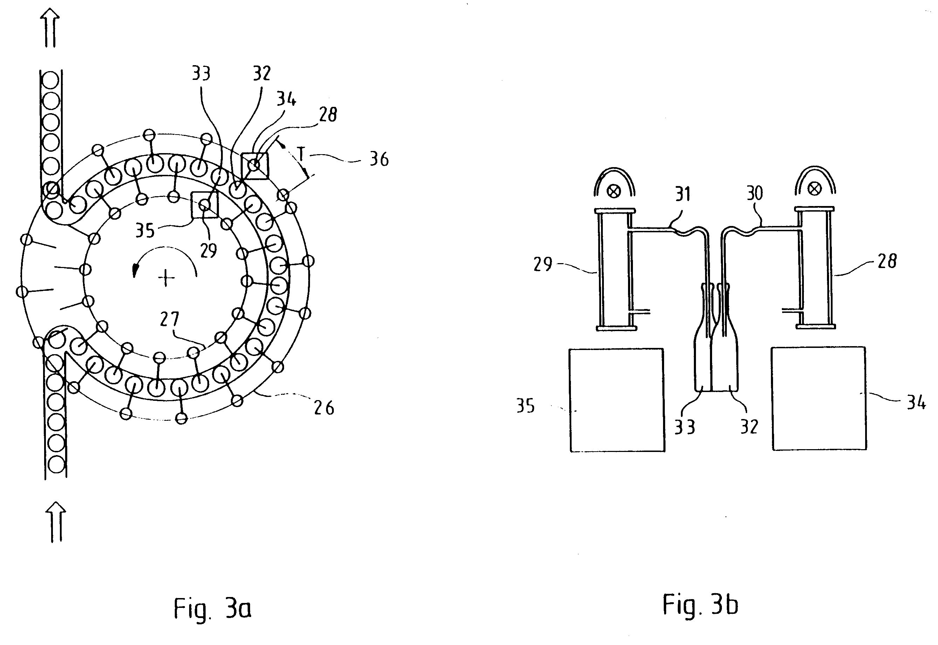

Um das Problem der kurzen Taktzeiten pro Flasche zu lösen, welches auf sehr großen Flaschendurchsätzen von bis zu 50.000 Flaschen pro Stunden beruht, werden in einer besonderen Ausbildung der Erfindung vorzugsweise mehr als ein Hochgeschwindigkeits-Detektorreihen-System gemäß Figur 2, sowie zur Reduktion der mechanischen Belastung und des mechanischen Verschleißes der Maschine das in Figur 3a und Figur 3b dargestellte System eingesetzt.

Die Gasproben der vom Transportsystem 11 gemäß Figur 2 ankommenden Flaschen 32, 33 werden über gleichlange, flexible Leitungen 30, 31 abwechselnd in die auf einer äußeren Bahn 26 und einer inneren Bahn 27 mitlaufenden Meßzellen 28, 29 gefüllt. Im Bereich der stationären Analysen-Module 34, 35 werden erfindungsgemäß die Meßzellen 28, 29 für die Meßzeit T 36 angehalten, um auch bei der hohen Taktrate eine ausreichende Analysenzeit sicherzustellen.

In bevorzugter Ausgestaltung kann vorgesehen sein, daß eine oder mehrere zusätzlich in den Strahlengang einschwenkbare Testzellen, die mit einem entsprechenden Testgas, wie Isopropylalkohol-, Methan-, Gasolin-, Ethanoldampf oder dergleichen definiert gefüllt sind, eine definierte durchstrahlte Weglänge und damit eine definierte Extinktion aufweisen, zur Überprüfung und Kalibrierung der Meßeinrichtung eingeschwenkt und Kalibrierungen durchgeführt werden. Alternativ kann vorgesehen sein, daß den für die Aufnahme des Gases an den Behältnissen vorgesehenen Untersuchungskammern entsprechende Kammern jeweils ein entsprechendes definiertes Testgas enthalten und zur Kalibrierung in den Förderer (im Austausch gegen die eigentliche Untersuchungskammer) eingestellt und durch die Meßoptik bewegt werden.

## Patentansprüche

1. Verfahren zum Untersuchen der Gasphasen in Behältern, wie in Getränkeflaschen, insbesondere zum Sortieren und Ausscheiden von Flaschen in einer Abfüllanlage für Getränkeflaschen, dadurch gekennzeichnet, daß eine aus den Flaschen entnommene Gasprobe mittels IR-Spektroskopie in einem Wellenlängenbereich von ca. 3,2 bis 3,6 »m spektroskopisch untersucht wird und der spektralzerlegte Strahl durch eine Reihe nebeneinander angeordneter Detektoren (Detektorreihe 20) detektiert wird.

2. Verfahren nach Anspruch 1, dadurch gekennzeichnet, daß die Behältnisse entsprechend den in Flaschen detektierten Stoffen auf mindestens zwei Transportwege aufgeteilt werden, wobei dem einen Transportweg Behältnisse zugeführt werden, die unbekannte bzw. undefinierbare Stoffe oder Giftstoffe enthalten und dem anderen Transportweg Behältnisse zugeführt werden, die ausschließlich Wasserdampf oder Luft enthalten.

3. Verfahren nach Anspruch 2, dadurch gekennzeichnet, daß dem ersten Transportweg Behältnisse zugeführt wurden, bei denen festgestellt wurde, daß sie unschädliche Gärprodukte oder Aromastoffe enthalten haben.

4. Verfahren nach Anspruch 3, dadurch gekennzeichnet, daß dem zweiten Transportweg Behältnisse zugeführt wurden, bei dem festgestellt wurde, daß sie unschädliche Gärprodukte oder Aromastoffe enthalten haben.

5. Verfahren nach einem der vorangehenden Ansprüche, dadurch gekennzeichnet, daß die Behältnisse "drei Transportwegen" für die Stoffklassen
- Giftstoffe sowie unbekannte und undefinierbare Produkte (2),
- unschädliche Gärprodukte und Aromastoffe (3, 4),
- Mineralwasserdampf, Luft (5)
zugeführt werden.

6. Verfahren nach einem der vorangehenden Ansprüche, dadurch gekennzeichnet, daß Giftstoffe oder unbekannte oder undefinierbare Stoffe enthaltende Flaschen ausgesondert und einer Entsorgung zugeführt werden, während Gärprodukte und/oder Aromastoffe enthaltende Flaschen einer Reinigung unterzogen und einer Süßgetränkefüllanlage zugeführt werden.

7. Verfahren nach einem der vorangehenden Ansprüche, dadurch gekennzeichnet, daß Behältnisse, die lediglich Mineralwasserdampf und/oder Luft enthalten haben, einer Füllanlage für Mineralwässer zugeleitet werden.

8. Verfahren nach einem der Ansprüche 1 bis 7, dadurch gekennzeichnet, daß eine spektrale Auflösung von besser als 0,02 »m gewählt wird.

9. Verfahren nach einem der Ansprüche 1 bis 8, dadurch gekennzeichnet, daß die Gasspektren mit einer Detektorreihe (20) aus HgCdTe- oder PbSe-Photoelementen erfaßt werden.

10. Verfahren nach einem der Ansprüche 1 bis 9, dadurch gekennzeichnet, daß die Detektorreihe (20) mindestens einstufig peltiergekühlt wird.

11. Verfahren nach einem der Ansprüche 1 bis 10, dadurch gekennzeichnet, daß die Substanzidentifikation und Substanzkonzentrationsbestimmung durch Echtzeit-Vergleich der jeweils gemessenen mit in einem Speicher befindlichen bekannten Spektren erfolgt.

12. Verfahren nach einem der Ansprüche 1 bis 11, dadurch gekennzeichnet, daß Einzeldetektoren (Pixel) der Detektorreihe (20) mittels einer mikromechanischen Verschiebeeinrichtung (24) periodisch längs der Erstreckungsrichtung der Detektorreihe auf Positionen zwischen die Ausgangspositionen zweier Einzeldetektoren gebracht werden.

13. Verfahren nach einem der Ansprüche 1 bis 12, dadurch gekennzeichnet, daß eine Wellenlängenkalibrierung der Detektorreihe (20) zyklisch durch Einbringen eines Testgases (49) in den Strahlengang (36, 50) erfolgt.

14. Verfahren nach einem der Ansprüche 1 bis 13, dadurch gekennzeichnet, daß der Partial-Dampfdruck der Gasphase (13) durch Erwärmung der Flaschenwand bzw. der an der Flaschenwand befindlichen Moleküle mit Infrarotstrahlung oder Mikrowellenstrahlung erhöht wird.

15. Verfahren nach einem der Ansprüche 1 bis 14, dadurch gekennzeichnet, daß die die Gasproben aufnehmenden Meßzellen auf einer äußeren und einer inneren Bahn, mitbewegt mit den Behältnissen, geführt werden.

16. Verfahren nach Anspruch 15, dadurch gekennzeichnet, daß die Gasproben ankommender Behältnisse (32, 33) über gleichlange, flexible Leitungen (30, 31) abwechselnd in die äußeren und inneren, mitlaufenden Meßzellen (28, 29) gefüllt werden.

17. Verfahren nach Anspruch 15 oder 16, dadurch gekennzeichnet, daß gleichzeitig jeweils der Gasinhalt der äußeren (28) und inneren Meßzelle (29) durch zwei Analysenstationen (34, 35) analysiert werden.

18. Verfahren nach einem der Ansprüche 1 bis 17, dadurch gekennzeichnet, daß die Meßzellen im Bereich der Analysenstationen für eine Meßzeit (T) angehalten werden, ohne daß der kontinuierliche Lauf des Flaschentransports beeinträchtigt wird.

19. Vorrichtung zum Untersuchen der Gasphasen in Behältnissen, wie in Getränkeflaschen, insbesondere zum Sortieren und Ausscheiden von Flaschen in einer Abfüllanlage für Getränkeflaschen, mit Meßzellen zur Aufnahme einer Gasprobe aus einem Behältnis und mit einem Spektrometer (19), in dem die die Gasprobe durchstrahlende Strahlung spektral zerlegt wird, gekennzeichnet durch eine Reihe von nebeneinander angeordneten, gleichzeitig von spektral zerlegtem Licht im IR-Wellenbereich von ca. 3,2 bis 3,6 »m durch eine Beleuchtungseinrichtung beleuchteten Detektoren (Detektorreihe 20).

20. Vorrichtung nach Anspruch 19, dadurch gekennzeichnet, daß hinter einer Untersuchungsstation für die Behältnisse zwei Transportwege vorgesehen sind, deren erstem Behältnisse, die unbekannte, undefinierbare oder Giftstoffe enthalten und deren zweitem Behältnisse, die Mineralwasserdampf oder Luft enthalten haben, zuführbar sind.

21. Vorrichtung nach Anspruch 20, dadurch gekennzeichnet, daß dem zweiten Transportweg Behältnisse zuführbar sind, die unschädliche Gärprodukte und Aromastoffe enthalten haben.

22. Vorrichtung nach einem der Ansprüche 19 bis 21, gekennzeichnet durch drei getrennte Transportwege für
- unbekannte, undefinierbare oder Giftstoffe (2),
- unschädliche Gärprodukte und Aromastoffe (3, 4),
- Mineralwasserdampf und Luft (5).

23. Vorrichtung nach einem der Ansprüche 19 bis 22, gekennzeichnet durch eine Einrichtung zur Einordnung in verschiedene Schadstoffklassen (2, 3, 4, 5) durch quantitative Messung der Infrarot-Absorption der jeweiligen Gasphasen.

24. Vorrichtung nach einem der Ansprüche 19 bis 23, dadurch gekennzeichnet, daß das spektrale Auflösungsvermögen des Infrarotspektrometers (19) besser als 0,02 »m ist.

25. Vorrichtung nach einem der Ansprüche 19 bis 24, dadurch gekennzeichnet, daß die Detektorreihe (20) HgCdTe- oder PbSe-Photoelemente aufweist.

26. Vorrichtung nach einem der Ansprüche 19 bis 25, gekennzeichnet durch eine mit der Detektorreihe (20) in thermischem Kontakt stehende Peltier-Kühleinrichtung (14).

27. Vorrichtung nach einem der Ansprüche 19 bis 26, gekennzeichnet durch eine Einrichtung zur Substanzidentifikation und Substanzkonzentrationsbestimmung durch Echtzeit-Vergleich der jeweils gemessenen mit in einem Speicher (23) befindlichen bekannten Spektren (Figur 3).

28. Vorrichtung nach einem der Ansprüche 19 bis 27, gekennzeichnet durch eine mikromechanische Verschiebeeinrichtung (24) zur Verschiebung der Einzeldetektoren (Pixel 43) der Detektorreihe (20) periodisch längs der Erstreckungsrichtung der Detektorreihe auf Positionen zwischen den Ausgangspositionen jeweils zweier Einzeldetektoren (43).

29. Vorrichtung nach einem der Ansprüche 19 bis 28, gekennzeichnet durch eine Einrichtung zur zyklischen Wellenlängenkalibrierung der Detektoren (20, 43) durch Einbringen eines Testgases (49) in den Strahlengang (16, 50).

30. Vorrichtung nach einem der Ansprüche 19 bis 29, gekennzeichnet durch eine Einrichtung zur Erhöhung des Partial-Dampfdrucks der Gasphase (13) mittels Erwärmung der Behälterwandungen mit Infrarotstrahlung (25) oder Mikrowellenstrahlung.

31. Vorrichtung nach einem der Ansprüche 19 bis 30, dadurch gekennzeichnet, daß die Meßzellen (28, 29) doppelt, nämlich auf einer äußeren Bahn (26) und einer inneren Bahn (27) angeordnet sind und mit den transportierten Behältnissen (32, 33) mitbewegbar sind.

32. Vorrichtung nach Anspruch 31, gekennzeichnet durch gleichlange, flexible Leitungen (30, 31) zur abwechselnden Füllung von Gasproben der ankommenden Behältnisse (32, 33) in die äußeren und inneren, mitlaufenden Meßzellen (28, 29).

33. Vorrichtung nach Anspruch 31 oder 32, dadurch gekennzeichnet, daß zwei Analysenstationen (34, 35) vorhanden sind, mittels derer gleichzeitig jeweils der Gasinhalt der äußeren Meßzelle (28) und der inneren Meßzelle (29) analysierbar ist.

34. Vorrichtung nach einem der Ansprüche 19 bis 33, dadurch gekennzeichnet, daß die Meßzellen (28, 29) im Bereich der Analysenstationen (34, 35) für eine Meßzeit (T (bei 36)) anhaltbar sind, ohne daß der kontinuierliche Lauf des Flaschentransports beeinträchtigt wird.

## Claims

1. Process for investigating gas phases in containers, such as drink bottles, particularly for sorting and extracting bottles in a bottling plant for drink bottles, characterized in that a gas sample removed from the bottles is spectroscopically investigated by means of IR spectroscopy in a wavelength range from approximately 3 2 to 3.6 »m and the spectrally dispersed beam is detected by a row of juxtaposed detectors (detector row 20).

2. Process according to claim 1, characterized in that the containers, corresponding to the substances detected in bottles, are subdivided over at least two conveying paths, in which the containers are supplied to one conveying path which contain the unknown or undefinable substances or toxic substances and containers are supplied to the other conveying path which exclusively contain water vapour or air.

3. Process according to claim 2, characterized in that to the first conveying path are supplied containers in which it has been established that they have contained harmless fermentation products or flavouring substances.

4. Process according to claim 3, characterized in that to the second conveying path are supplied containers with respect to which it has been established that they have contained harmless fermentation products or flavouring substances.

5. Process according to one of the preceding claims, characterized in that the containers are supplied to "three conveying paths" for the substance classes:
- toxic substances, as well as unknown and undefinable products (2),
- harmless fermentation products and flavouring substances (3, 4),
- mineral water vapour and air (5).

6. Process according to one of the preceding claims, characterized in that the bottles containing toxic substances or unknown or undefinable substances are extracted and disposed of, whereas bottles containing fermentation products and/or flavouring substances are cleaned and supplied to a soft drinks bottling plant.

7. Process according to one of the preceding claims, characterized in that containers, which have only contained mineral water vapour and/or air are supplied to a mineral water bottling plant.

8. Process according to one of the claims 1 to 7, characterized in that a spectral resolution of better than 0.02 »m is selected.

9. Process according to one of the claims 1 to 8, characterized in that the gas spectra are detected with a detector row (20) of HgCdTe or PbSe photocells.

10. Process according to one of the claims 1 to 9, characterized in that the detector row (20) is Peltier cooled in at least one stage.

11. Process according to one of the claims 1 to 10, characterized in that the substance identification and substance concentration determination take place by real time comparison of the in each case measured spectra with known spectra in a memory.

12. Process according to one of the claims 1 to 11, characterized in that the individual detectors (pixels) of the detector row (20) are brought by means of a micromechanical displacement device (24) periodically along the extension direction of the detector row to positions between the starting positions of two individual detectors.

13. Process according to one of the claims 1 to 12, characterized in that a wavelength calibration of the detector row (20) takes place cylically by introducing a test gas (49) into the optical path (36, 50).

14. Process according to one of the claims 1 to 13, characterized in that the partial vapour pressure of the gas phase (13) is increased by heating the bottle wall or the molecules located on the bottle wall with infrared radiation or microwave radiation.

15. Process according to one of the claims 1 to 12, characterized in that the measuring cells receiving the gas samples are moved together with the containers on an outer and an inner path.

16. Process according to claim 15, characterized in that the gas samples of incoming containers (32, 33) are filled by means of equal length, flexible lines (30, 31) alternately into the outer and inner, also revolving measuring cells (28, 29).

17. Process according to claim 15 or 16, characterized in that simultaneously in each case the gas content of the outer (28) and inner (29) measuring cell is analyzed by two analytical stations (34, 35).

18. Process according to one of the claims 1 to 17, characterized in that the measuring cells are stopped in the vicinity of the analytical stations for a measuring time (T) without the continuous run of the bottle conveying being impaired.

19. Apparatus for investigating gas phases in containers, such as drink bottles, particularly for sorting and extracting bottles in a bottling plant for drink bottles, with measuring cells for receiving a gas sample from a container and with a spectrometer (19) in which the radiation irradiating the gas sample is spectrally dispersed, characterized by a row of juxtaposed detectors (detector row 20) simultaneously illuminated by an illuminating device with spectrally dispersed light in the IR wavelength range of approximately 3 2 to 3 6 »m.

20. Apparatus according to claim 19, characterized in that behind an investigating station for the containers are provided two conveying paths, to the first of which can be supplied containers containing unknown, undefinable or toxic substances and to the second are suppliable containers which have contained mineral water vapour or air.

21. Apparatus according to claim 20, characterized in that to the second conveying path can be supplied containers which have contained harmless fermentation products or flavouring substances.

22. Apparatus according to one of the claims 19 to 21, characterized by three separate conveying paths for
- unknown, undefinable or toxic substances (2),
- harmless fermentation products and flavouring substances (3, 4),
- mineral water vapour and air (5).

23. Apparatus according to one of the claims 19 to 22, characterized by a device for placing in different harmful substance classes (2, 3, 4, 5) by the quantitative measurement of the infrared absorption of the particular gas phases.

24. Apparatus according to one of the claims 19 to 23, characterized in that the spectral resolving power of the infrared spectrometer (19) is better than 0.02 »m.

25. Apparatus according to one of the claims 19 to 24, characterized in that the detector row (20) has HgCdTe or PbSe photocells.

26. Apparatus according to one of the claims 19 to 25, characterized by a Peltier cooling device (14) in thermal contact with the detector row (20).

27. Apparatus according to one of the claims 19 to 26, characterized by a device for substance identification and substance concentration determination by real time comparison of the particular spectra measured with spectra stored in a memory (23) (fig. 3).

28. Apparatus according to one of the claims 19 to 27, characterized by a micromechanical displacement device (24) for displacing the individual detectors (pixels 43) of the detector row (20) in periodic manner along the extension direction of the detector rows to positions between the starting positions of in each case two individual detectors (43).

29. Apparatus according to one of the claims 19 to 28, characterized by a device for the cyclic wavelength calibration of the detectors (20, 43) by introducing a test gas (49) into the optical path (16, 50).

30. Apparatus according to one of the claims 19 to 29, characterized by a device for increasing the partial vapour pressure of the gas phase (13) by heating the container walls with infrared radiation (25) or microwave radiation.

31. Apparatus according to one of the claims 19 to 30, characterized in that the measuring cells (28, 29) are arranged in double form, namely on an outer path (26) and on an inner path (27) and are jointly movable with the conveyed containers (32, 33).

32. Apparatus according to claim 31, characterized by equal length, flexible lines (30, 31) for the alternate filling of gas samples of the incoming containers (32, 33) into the outer and inner, revolving measuring cells (28, 29).

33. Apparatus according to claim 31 or 32, characterized in that there are two analytical stations (34, 35), by means of which simultaneously it is possible to analyze the gas content of the outer measuring cell (28) and the inner measuring cell (29).

34. Apparatus according to one of the claims 19 to 33, characterized in that the measuring cells (28, 29) can be stopped in the vicinity of the analytical stations (34, 35) for a measuring time (T at 36), without impairing the continuous run of the bottle conveying.

## Revendications

1. Procédé pour analyser les phases gazeuses dans des récipients, comme par exemple des bouteilles à boissons, en particulier pour classer et rejeter des bouteilles dans une installation de remplissage pour bouteilles à boissons, caractérisé en ce qu'un échantillon de gaz prélevé sur les bouteilles est analysé spectroscopiquement par spectroscopie infrarouge dans un domaine de longueurs d'onde d'environ 3,2 à 3,6 »m et en ce que le rayon décomposé spectralement est détecté par une série de détecteurs juxtaposés (série de détecteurs 20).

2. Procédé selon la revendication 1, caractérisé en ce que les récipients sont répartis, en fonction des substances détectées dans des bouteilles, sur au moins deux voies de transport, de telle façon qu'à l'une des voies de transport soient amenés des récipients qui contiennent des substances inconnues ou indéfinissables ou des substances toxiques et qu'à l'autre voie de transport soient amenés des récipients qui contiennent exclusivement de la vapeur d'eau ou de l'air.

3. Procédé selon la revendication 2, caractérisé en ce qu'à la première voie de transport sont amenés des récipients pour lesquels il a été établi qu'ils contiennent des produits de fermentation ou aromates inoffensifs.

4. Procédé selon la revendication 3, caractérisé en ce quà la seconde voie de transport sont amenés des récipients pour lesquels il a été établi qu'ils ont contenu des produits de fermentation ou aromates inoffensifs.

5. Procédé selon l'une quelconque des revendications précédentes, caractérisé en ce que les récipients sont amenés à trois voies de transport pour les catégories de substances suivantes :
- substances toxiques ainsi que produits inconnus et indéfinissables (2) ;
- produits de fermentation et aromates inoffensifs (3, 4) ; et
- vapeur d'eau minérale et de l'air (5).

6. Procédé selon l'une quelconque des revendications précédentes, caractérisé en ce que des bouteilles contenant des substances toxiques ou des substances inconnues ou indéfinissables sont séparées et amenées à une dépollution, alors que des bouteilles contenant des produits de fermentation et/ou des aromates sont soumises à un nettoyage et amenées à une installation de remplissage avec des boissons douces.

7. Procédé selon l'une quelconque des revendications précédentes, caractérisé en ce que des récipients qui n'ont contenu que de la vapeur d'eau minérale et/ou de l'air sont dirigées vers une installation de remplissage pour de l'eau minérale.

8. Procédé selon l'une quelconque des revendications précédentes, caractérisé en ce qu'il est choisi une résolution spectrale meilleure que 0,02 »m.

9. Procédé selon l'une quelconque des revendications précédentes, caractérisé en ce que les spectres des gaz sont déterminés au moyen d'une série de détecteurs (20) constitués par des cellules photoélectriques en HgCdTe ou PbSe.

10. Procédé selon l'une quelconque des revendications précédentes, caractérisé en ce que la série de détecteurs (20) est refroidie à au moins un étage par des éléments Peltier.

11. Procédé selon l'une quelconque des revendications précédentes, caractérisé en ce que l'identification et la détermination de la concentration de substances se réalisent par comparaison en temps réel des spectres mesurés à chaque fois avec des spectres connus se trouvant dans une mémoire.

12. Procédé selon l'une quelconque des revendications précédentes, caractérisé en ce que les détecteurs individuels (pixels) de la série de détecteurs (20) sont amenés, au moyen d'un dispositif de déplacement micromécanique (24), périodiquement dans la direction de l'étendue de la série de détecteurs, à des positions situées entre les positions de départ de deux détecteurs individuels.

13. Procédé selon l'une quelconque des revendications précédentes, caractérisé en ce qu'il s'effectue cycliquement un calibrage des longueurs d'onde de la série de détecteurs (20) par l'introduction d'un gaz d'essai (49) dans le trajet des rayons (16, 50).

14. Procédé selon l'une quelconque des revendications précédentes, caractérisé en ce que la pression de vapeur partielle de la phase gazeuse (13) est augmentée par réchauffement de la paroi des bouteilles, c'est-à-dire des molécules se trouvant dans la paroi de la bouteille, au moyen d'un rayonnement infrarouge ou d'un rayonnement à micro-ondes.

15. Procédé selon l'une quelconque des revendications précédentes, caractérisé en ce que les cellules de mesure recevant les échantillons de gaz sont guidées sur une voie extérieure et une voie intérieure, en étant déplacées avec les récipients.

16. Procédé selon la revendication 15, caractérisé en ce que les échantillons de gaz de récipients (32, 33) qui arrivent, sont introduits, par l'intermédiaire de tuyaux flexibles (30, 31) d'une même longueur, alternativement dans les cellules de mesure extérieures et intérieures (28, 29) se déplaçant avec les récipients.

17. Procédé selon la revendication 15 ou 16, caractérisé en ce que le gaz contenu dans la cellule de mesure extérieure (28) et le gaz contenu dans la cellule de mesure intérieure (29) sont chaque fois analysés simultanément par deux postes d'analyse (34, 35).

18. Procédé selon l'une quelconque des revendications précédentes, caractérisé en ce que les cellules de mesure sont arrêtées dans la région des postes d'analyse, pendant une durée de mesure (T) sans porter préjudice au processus continuel du transport de bouteilles.

19. Dispositif pour analyser les phases gazeuses dans des récipients, comme par exemple des bouteilles à boissons, en particulier pour classer et rejeter des bouteilles dans une installation de remplissage pour bouteilles à boissons, équipé de cellules de mesure pour recevoir un échantillon de gaz prélevé sur un récipient, et d'un spectromètre (19) dans lequel le rayonnement passant à travers l'échantillon de gaz est décomposé spectralement, caractérisé par une série de détecteurs juxtaposés (série de détecteurs 20) éclairés simultanément, au moyen d'un appareil d'éclairement, par de la lumière, décomposée spectralement, dans la plage d'ondes infrarouges d'environ 3,2 à 3,6 »m.

20. Dispositif selon la revendication 19, caractérisé en ce que derrière un poste d'analyse pour les récipients sont prévues deux voies de transport à la première desquelles peuvent être amenés des récipients qui ont contenu des substances inconnues, indéfinissables ou toxiques et à la seconde desquelles peuvent être amenés des récipients qui ont contenu de la vapeur d'eau minérale ou de l'air.

21. Dispositif selon la revendication 20, caractérisé en ce qu'à la seconde voie de transport peuvent être amenés des récipients qui ont contenu des produits de fermentation et aromates inoffensifs.

22. Dispositif selon l'une quelconque des revendications 19 à 21, caractérisé par trois voies de transport séparées pour:
- substances inconnues, indéfinissables ou toxiques (2);
- produits de fermentation et aromates inoffensifs (3, 4) ; et
- vapeur d'eau minérale et air (5).

23. Dispositif selon l'une quelconque des revendications 19 à 22, caractérisé par un équipement pour classer dans différentes classes de substances nocives (2, 3, 4, 5) en mesurant quantitativement l'absorption d'infrarouge par les phases gazeuses respectives.

24. Dispositif selon l'une quelconque des revendications 19 à 23, caractérisé en ce que le pouvoir de résolution spectrale du spectromètre infrarouge (19) est meilleur que 0,02 »m.

25. Dispositif selon l'une quelconque des revendications 19 à 24, caractérisé en ce que la série de détecteurs (20) comporte des cellules photoélectriques en HgCdTe ou PbSe.

26. Dispositif selon l'une quelconque des revendications 19 à 25, caractérisé par un appareil de refroidissement Peltier (14) se trouvant en contact thermique avec la série de détecteurs (20).

27. Dispositif selon l'une quelconque des revendications 19 à 26, caractérisé par un appareil pour identifier et déterminer la concentration de substances par une comparaison en temps réel des spectres mesurés à chaque fois avec des spectres (figure 3) connus se trouvant dans une mémoire (23).

28. Dispositif selon l'une quelconque des revendications 19 à 27, caractérisé par un appareil de déplacement micromécanique (24) pour faire passer les détecteurs individuels (pixels 43) de la série de détecteurs (20) périodiquement, dans la direction de l'étendue de la série de détecteurs, à des positions situées entre les positions de départ respectives de deux détecteurs individuels (43).

29. Dispositif selon l'une quelconque des revendications 19 à 28, caractérisé par un équipement pour calibrer les longueurs d'onde cycliques des détecteurs (20, 43) par introduction d'un gaz d'essai (49) dans le trajet des rayons (16, 50).

30. Dispositif selon l'une quelconque des revendications 19 à 29, caractérisé par un équipement pour accroître la pression de vapeur partielle de la phase gazeuse (13) par réchauffement des parois des récipients au moyen d'un rayonnement infrarouge (25) ou d'un rayonnement à micro-ondes.

31. Dispositif selon l'une quelconque des revendications 19 à 30, caractérisé en ce que les cellules de mesure (28, 29) sont disposées en double, à savoir sur une voie extérieure (26) et une voie intérieure (27) et peuvent être déplacées conjointement avec les récipients transportés (32, 33).

32. Dispositif selon la revendication 31, caractérisé par des tuyaux flexibles (30, 31) de même longueur pour introduire alternativement des échantillons de gaz des récipients amenés (32, 33) dans les cellules de mesure extérieures et intérieures (28, 29) se déplaçant avec les récipients.

33. Dispositif selon la revendication 31 ou 32, caractérisé en ce qu'il est prévu deux postes d'analyse (34, 35) au moyen desquels peut chaque fois être analysé simultanément le contenu gazeux de la cellule de mesure extérieure (28) et de la cellule de mesure intérieure (29).

34. Dispositif selon l'une quelconque des revendications 19 à 33, caractérisé en ce que les cellules de mesure (28, 29) dans la région des postes d'analyse (34, 35) peuvent être arrêtées pendant une durée de mesure T (en 36)) sans porter préjudice au défilement continuel du transport de bouteilles.
